(19) Europäisches Patentamt

European Patent Office

Office européen des brevets

(11) **EP 0 631 757 B1**

(12) **EUROPÄISCHE PATENTSCHRIFT**

(45) Veröffentlichungstag und Bekanntmachung des Hinweises auf die Patenterteilung:
**25.08.1999 Patentblatt 1999/34**

(51) Int. Cl.$^6$: **A61B 5/026**, A61B 3/12

(21) Anmeldenummer: 94108430.3

(22) Anmeldetag: **01.06.1994**

(54) **Verfahren und Gerät zur Messung der Fliessgeschwindigkeit, insbesondere des Blutes**

Method and device for measuring the flow velocity particularly of the blood

Méthode et dispositif pour mesurer la vitesse d'écoulement notamment de sang

(84) Benannte Vertragsstaaten:
**AT BE CH DE DK ES FR GB IT LI NL SE**

(30) Priorität: **02.07.1993 DE 4322043**

(43) Veröffentlichungstag der Anmeldung:
**04.01.1995 Patentblatt 1995/01**

(73) Patentinhaber:
**HEIDELBERG ENGINEERING OPTISCHE MESSSYSTEME GmbH
69120 Heidelberg (DE)**

(72) Erfinder:
• **Michelson, Georg, Dr.
D-91083 Baiersdorf-Hagenau (DE)**
• **Zinser, Gerhard, Dr.
D-67346 Speyer (DE)**
• **Schmauss, Bernhard
D-91054 Erlangen (DE)**

(74) Vertreter:
**Klose, Hans, Dipl.-Phys. et al
Rechts- und Patentanwälte,
Reble & Klose,
Bereich Patente & Marken,
Postfach 12 15 19
68066 Mannheim (DE)**

(56) Entgegenhaltungen:
EP-A- 0 282 210       EP-A- 0 376 470
EP-A- 0 488 614       EP-A- 0 498 280
WO-A-92/03084

• PATENT ABSTRACTS OF JAPAN vol. 13, no. 224 (C-599) (3572) 24. Mai 1989 & JP-A-01 037 931 (HITOSHI FUJII) 8. Februar 1989

**Beschreibung**

Die Erfindung betrifft ein Verfahren gemäß den im Oberbegriff des Patentanspruchs 1 angegebenen Merkmalen. Ferner betrifft die Erfindung ein Gerät gemäß den im Oberbegriff des Patentanspruchs 8 angegebenen Merkmalen.

Aus der EP-A-0 282 210 ist ein Verfahren sowie ein Gerät der genannten Art zur Messung der Fließgeschwindigkeit des Blutes unter Ausnutzung des Doppler-Effekts bekannt. Hierbei wird mittels eines Laserstrahls eine zweidimensionale rasterförmige Abtastung durchgeführt. Zur Auffächerung des Laserstrahls in eine Linie ist eine Zylinderlinse vorgesehen und mittels eines rotierenden Spiegels wird der linienförmige Strahl über das Meßfeld bewegt. Das vom Meßfeld reflektierte Licht wird über den genannten Spiegel sowie eine Optik auf eine Reihe von Meßzellen projiziert, welche von der jeweiligen Lichtintensität abhängige Signale liefern. Der Auflösung eines derartigen Systems sind insofern Grenzen gesetzt, als die in einer Reihe nebeneinander angeordneten Meßzellen, wie Fotodioden oder ähnliches, eine vergleichsweise große Breite aufweisen. Gefäße mit kleinen Abmessungen, wie insbesondere Kapillare der Netzhaut eines Auges können mit dem vorbekannten Verfahren bzw. dem vorbekannten Gerät nicht ohne weiteres detektiert werden. Ferner wird der linienförmige Lichtstrahl entsprechend der Drehzahl des genannten Spiegels über das Meßfeld bewegt, wobei unter Berücksichtigung der nachfolgenden Digitalisierung eine Linie nach der anderen des Meßfeldes abgetastet wird.

Ferner sind aus der EP-A-488 614 sowie der PCT-Anmeldung WO-A-92/03084 Vorrichtungen zur Messung der Fließgeschwindigkeit des Blutes bekannt, enthaltend ein Abtastsystem zur zweidimensionalen Abtastung. Hierbei erfolgt die Abtastung Zeile pro Zeile nacheinander, wobei beim Erreichen des letzten Punktes einer Zeile jeweils auf die nächstfolgende Zeile gesprungen wird. Die Steuerung erfolgt mittels einer Kontrolleinheit, wobei ferner eine Umwandlung der mittels eines Detektors erfaßten analogen Meßwerte in digitale Werte erfolgt.

Des weiteren ist aus der PCT-Anmeldung WO-A-93 03 676 ein Verfahren sowie ein Gerät der eingangs genannten Art zur Messung der Fließgeschwindigkeit einer Flüssigkeit, insbesondere des Blutes durch Bestimmung der Frequenzverschiebung eines in der Flüssigkeit reflektierten Laserstrahls gemäß des optischen Doppler-Effektes bekannt. Auch dieses Meßverfahren ist ebenso wie die eingangs erläuterten Verfahren nicht invasiv, doch es ermöglicht die Messung der Fließgeschwindigkeit nur an einer Stelle. Ferner ist ein hoher Aufwand seitens des Patienten und des Untersuchenden erforderlich, zumal der Zielstrahl für längere Zeit exakt auf einen bestimmten Punkt gerichtet sein muß. Mit diesem Verfahren ist es bisher nicht möglich, insbesondere in der Augenheilkunde den Blutfluß im Gefäßsystem der Aderhaut zu vermessen. Des weiteren ist aus dem US-Patent 4 142 796 ein auf dem optischen Doppler-Effekt beruhendes Verfahren sowie eine entsprechende Vorrichtung zur Diagnostik in der Augenheilkunde bekannt.

In vielen Bereichen der medizinischen Diagnostik und Therapie ist die Messung von Blutflußgeschwindigkeiten erforderlich. Insbesondere besteht in der Augenheilkunde eine große klinische Notwendigkeit für eine örtlich aufgelöste und kontinuierliche Messung des Blutflusses in der Netzhaut. Bekanntlich werden die drei Zellschichten der Netzhaut durch zwei unabhängige Blutgefäßsysteme mit Sauerstoff versorgt. Die Fotorezeptoren der untersten Schicht werden von der Aderhaut, die Bipolar-Zellen/Amakrin-Zellen sowie die Nervenzellen der obersten Schicht werden vom intraretinalen Gefäßbett mit versorgt. Es ist erforderlich, die Fließgeschwindigkeiten in den zuführenden Arteriolen, den retinalen Kapillaren und in dem arterio-venösen Shunt-Gefäßen, welche unter Umgehung der Kapillaren den Blutfluß direkt in die Venolen ableiten, zu messen.

Außer der eingangs erwähnten Laser-Doppler-Velocimetrie gelangen noch zwei weitere Verfahren zur Bestimmung der retinalen Durchblutung zur klinischen Anwendung. So beruht die sehr häufig durchgeführte Fluoreszenzangiographie auf einer qualitativen Beurteilung der retinalen Arteriolen und Kapillaren nach intravenöser Injektion von Fluoreszenz-Farbstoff. Die quantitative Auswertung von digitalisierten Fluoreszenz-Bildern ermöglicht Aussagen über spezielle Füllzeiten wie Arm-Retina-Zeit oder arterio-venöse Passagezeit. Die intravenöse Injektion eines Fluoreszenz-Farbstoffes stellt jedoch ein invasives Verfahren dar, welches ein Restrisiko eines anaphylaktischen Schocks beinhaltet und daher vorwiegend in Augenkliniken durchgeführt wird. Die Fluoreszenzangiographie ist ein zweidimensional örtlich auflösendes Verfahren, jedoch nicht zeitlich auflösend und invasiv mit vitalem Rest-Risiko. Schließlich kann mittels der nicht-invasiven Ultraschall-Doppler-Sonographie (Duplex-Sonographie, gepulste Doppler-Sonographie) an begrenzten Stellen zeitlich aufgelöst die Blußflußgeschwindigkeit in orbitalen Arterien, Arteriolen und Venolen bis ca. 1 mm Durchmesser bestimmt werden. Hingegen sind kleinere Gefäße und Kapillaren der Netzhaut mit dieser Methode nicht detektierbar.

Aus der EP-A-0 498 280 ist eine Vorrichtung zum Abtasten eines Ojektes mit einem Strahlenbündel in zwei im wesentlichen orthogonalen Richtungen bekannt. Diese Vorrichtung enthält einen ersten und einen zweiten Scanner mit jeweils einem Spiegel, deren Drehachsen in zueinander orthogonalen Ebenen verlaufen. Diese Vorrichtung weist eine kompakte Bauweise auf und enthält im Strahlengang zwischen den genannten Spiegeln kein zusätzliches optisches System. Der Spiegel des ersten Scanners ist in einem bestimmten Abstand von seiner Drehachse angebracht. Ein optisch einwandfreier Abtastvorgang wird vor allem dadurch gewährleistet, daß der Mittelpunkt des Spiegels des zweiten Scanners in der Mitte des genannten Abstandes zwischen der Drehachse und dem Spiegel des ersten Scanners angeordnet ist, wobei das Strahlenbündel vom Spiegel des ersten Scanners direkt zum Spiegel des zweiten Scanners verläuft.

Ferner ist aus der Publikation J. Phys. E: Sci. Instrum. Vol. 17 (1984), 131-136, ein Windgeschwindigkeitsmesser bekannt, in welchem unter Nutzung des Doppler-Effekts und Abtastung mittels eines Laserstrahls die Windgeschwindigkeit beispielsweise in einem Windkanal gemessen werden kann. Des weiteren ist aus der Publikation Rev. Sci. Instrum. 52 (11) (1981), 1676-1681, ein Laser-Doppler-System bekannt, mit welchem gleichfalls vor allem die Windgeschwindigkeit gemessen werden kann. Bei derartigen Geräten oder Systemen sind besondere, starr angeordnete Spiegel vorhanden und insgesamt ist ein vergleichsweise großes Bauvolumen erforderlich und der Einsatz in der medizinischen Diagnostik und Therapie ist nicht ohne weiteres möglich.

Hiervon ausgehend liegt der Erfindung die Aufgabe zugrunde, ein Verfahren sowie ein Gerät zur Durchführung des Verfahrens vorzuschlagen, um eine räumlich und zeitlich aufgelöste Messung von Fließgeschwindigkeiten eines strömenden Mediums zuverlässig zu ermöglichen. Das Verfahren und ebenso das zur Durchführung vorgeschlagene Gerät sollen eine hohe Meßgenauigkeit ergeben.

Die Lösung dieser Aufgabe erfolgt gemäß den Merkmalen der Patentansprüche 1 und 8.

Das erfindungsgemäße Verfahren beinhaltet die Kombination der Messung einerseits der Fließgeschwindigkeit mittels des optischen Doppler-Effektes und andererseits der Laserscanning Technik, wobei durch die Vorgabe des Abtastvorganges sowie die hochempfindliche Detektion der erforderliche Meßbereich abgedeckt und die geforderte Meßgenauigkeit erreicht wird. Im Gegensatz zu bekannten Verfahren erfolgt die Messung der Fließgeschwindigkeit der Flüssigkeit gleichzeitig dreidimensional örtlich auflösend, zeitlich auflösend, nicht-invasiv und schnell. Das zu untersuchende Objekt, beispielsweise die Netzhaut, wird mittels des Laserstrahls zweidimensional rasterförmig abgetastet, wobei an jedem Punkt durch wiederholtes Abtasten das reflektierte Licht in schneller Folge mehrfach gemessen wird. Aus der zeitlichen Variation der gemessenen Intensität des am jeweiligen Abtastpunkt reflektierten Lichts wird die Doppler-Verschiebung berechnet und hieraus die Fließgeschwindigkeit, insbesondere des Blutes, an jedem Punkt bestimmt. Es ergibt sich eine in zwei Dimensionen örtlich aufgelöste Abbildung des retinalen Blutflusses. Durch Wiederholen der beschriebenen Messung ergibt sich darüber hinaus ein zeitlich aufgelöstet Bild des retinalen Blutflusses. Durch Realisation des Laserscanning-Systems, insbesondere in konfokaler Anordnung, ergibt sich zudem eine Ortsauflösung in der Tiefe, so daß die Messungen selektiv in einzelnen Schichten des Objektes erfolgen können. Durch angepaßte Vorgabe der Wellenlänge des Lasers können somit bei der Untersuchung der Netzhaut das intraretinale Gefäßbett und die Aderhautgefäße voneinander getrennt vermessen werden.

Das erfindungsgemäße Scanning-Laser-Doppler-Velocimetrie-Verfahren kann sowohl außerhalb auch innerhalb des medizinischen Bereiches zum Einsatz gelangen. Grundsätzlich erstreckt sich der Anwendungsbereich des Verfahrens über alle Gebiete, die eine räumlich aufgelöste Messung von Fließgeschwindigkeiten eines strömenden Mediums erfordern. Die Einsatzmöglichkeiten des Verfahrens erstrecken sich in der Augenheilkunde auf alle Gebiete, in denen heute die invasive Fluoreszenzangiographie zum Einsatz gelangt und kann diese ersetzen. Das Verfahren ist nicht-invasiv und erfordert keine Erweitung der Pupille des untersuchten Auges. Ferner ermöglicht das Verfahren die zeitlich und dreidimensional örtlich aufgelöste Messung des retinalen Blutflusses. Weitere typische Anwendungen des Verfahrens sind die Messungen der Regulationsfähigkeit des retinalen Gefäßsystems sowie die Bestimmung der retinalen Durchblutungsverhältnisse bei der Glaukomerkrankung. Medizinische Einsatzgebiete außerhalb der Augenheilkunde bilden vor allem die örtlich aufgelöste Blutflußbestimmung der Haut sowie weiterer Organe wie Herz, Leber, Darm und Gehirn im intraoperativen Einsatz.

Das erfindungsgemäße Gerät zur Durchführung des Verfahrens enthält ein Laser-Scanning-System, eine Kontroll- und Steuerelektronik für den Abtastvorgang und für die Gewinnung der Daten sowie einen Computer zur Analyse der gewonnenen Daten. Das Laser-Scanning-System enthält eine Laserquelle, eine Strahlablenkungseinheit zur periodischen Ablenkung des Laserstrahls in zwei zueinander senkrechten Richtungen und eine Abbildungsoptik zur Abbildung des abtastenden Laserstrahls auf das zu untersuchende Objekt. Ferner ist ein Fokusierelement zur Bewegung der Fokalebene, ein Auskoppelelement zur Trennung des reflektierten Lichtstrahls vom beleuchtenden Laserstrahls sowie eine Detektionseinrichtung zur Messung der Intensität des reflektierten Lichtstrahles vorgesehen. In der Augenheilkunde bei der Untersuchung der Netzhaut sind die lichtbrechenden Medien des Auges selbst Bestandteil der Abbildungsoptik, mittels welcher der abtastende Laserstrahl auf das zu untersuchende Objekt abgebildet wird. Die erwähnte Strahlablenkungseinheit sowie das Fokusierelement können insbesondere als die in der eingangs erwähnten DE 4103298 C2 beschriebene Vorrichtung zum Abtasten eines Objektes ausgebildet sein.

Weitere besondere Ausgestaltungen und Vorteile sind in den Unteransprüchen und der nachfolgenden Beschreibung eines Ausführungsbeispiels angegeben.

Die Erfindung wird nachfolgend anhand der Zeichnung näher erläutert. Es zeigen:

Fig. 1     eine Prinzipdarstellung des Gerätes zur Durchführung des Verfahrens,

Fig. 2     eine Prinzipdarstellung des Gerätes gemäß Fig. 1 in Blickrichtung II,

Fig. 3     den grundsätzlichen Aufbau des erfindungsgemäßen Gerätes.

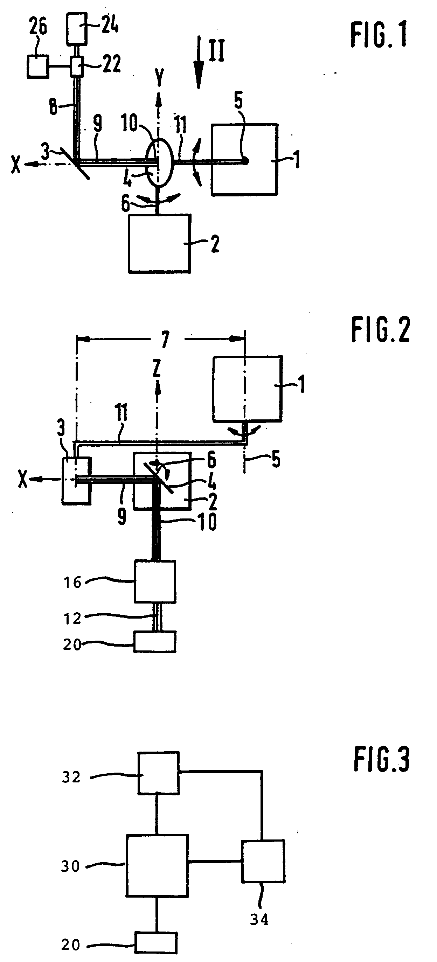

Fig. 1 zeigt schematisch einen ersten Scanner 1 und einen zweiten Scanner 2. Mit dem ersten Scanner 1 ist ein erster Spiegel 3 mechanisch gekopppelt und um eine zur Zeichenebene senkrechte Achse 5 schwenkbar angeordnet. Dem zweiten Scanner 2 ist ein zweiter Spiegel 4 zugeordnet, welcher um eine zur Zeichenebene parallele Achse 6 schwenkbar ist. Die Achsen 5 und 6 verlaufen in zueinander orthogonalen Ebenen und stehen senkrecht zueinander. Wesentlich ist, daß der erste Spiegel 3 bezüglich des Scanners 1 in einem Abstand 7 angeordnet ist und ferner die Drehachse 6 des Scanners 2 in der Mitte zwischen der Drehachse des Scanners 1 und dem Spiegel 3 verläuft. Das Zentrum des zweiten Spiegels 4 befindet sich erfindungsgemäß auf dem halben Abstand zwischen dem Zentrum des ersten Spiegels 3 und der Drehachse 5 des ersten Scanners 1. Die Drehachse 5 des ersten Spiegels 3 verläuft im wesentlichen parallel zur Spiegelebene, wobei jedoch der Abstand 7 zwischen dem Zentrum des ersten Spiegels 3 und der Drehachse 5 vorhanden ist. Auch die Drehachse 6 des zweiten Spiegels 4 verläuft im wesentlichen parallel zu diesem. Die Drehachse 6 verläuft ferner im wesentlichen durch das Zentrum des zweiten Spiegels, wobei jedoch bedarfsweise auch ein Abstand vorgegeben werden kann. Maßgebend ist, daß das Zentrum des zweiten Spiegels um den halben Abstand 7 von der Drehachse 5 angeordnet ist.

Zwischen den beiden Spiegeln 3 und 4 sind keine Linsen oder optische Abbildungsmittel vorhanden und innerhalb der Vorrichtung gelangen somit die Strahlen 9 direkt von dem einen Spiegel zum anderen. Schematisch sind Strahlen 8 angedeutet, welche von bzw. zu einer Einrichtung 22 zur Auskopplung der Strahlen gelangen. Die Strahlen einer Strahlenquelle 24, insbesondere eines Lasers, gelangen zunächst zu dieser Einrichtung und von dort zu der Vorrichtung gemäß Fig. 1 und weiter zu dem Objekt. Die vom Objekt reflektierten Strahlen gelangen wiederum über die erfindungsgemäße Vorrichtung und von dieser zu der Auskopplungseinrichtung 22, welche sie dann einer Auswerteinrichtung bzw. einem Detektor 26 bevorzugt eine Avalanche-Fotodiode zur weiteren Auswertung zuführt. Die zum Objekt ausgesendeten bzw. vom Objekt reflektierten Strahlen 10 verlaufen im wesentlichen orthogonal zu der Ebene, in welcher die Strahlen 8 und 9 verlaufen.

Es sei davon ausgegangen, daß ein orthogonales Achsensystem mit seinem Ursprungspunkt im Zentrum des zweiten Spiegels 4 liegt, wobei die X-Y-Ebene mit der Zeichenebene übereinstimmt. Die Strahlen 8 verlaufen parallel zur Y-Richtung, während die Strahlen 9 zwischen den beiden Spiegeln 3, 4 entlang der X-Richtung verlaufen. Die Drehachse 6 des zweiten Scanners liegt in der Y-Richtung, während die Drehachse 5 orthogonal zur X-Y-Ebene steht.

Fig. 2 zeigt eine Ansicht des Gerätes aus Fig. 1 in Blickrichtung II, wobei die Achse 5 in der Zeichenebene und die Achse 6 senkrecht zur Zeichenebene verlaufen. Der Spiegel 3 ist mit dem Scanner 1 über einen Arm 11 verbunden und weist somit zur Drehachse 5 den Abstand 7 auf. Die Zeichenebene entspricht der X-Z-Ebene des Koordinatensystems mit Ursprung im Zentrum des zweiten Spiegels 4. Die vom zweiten Spiegel 4 reflektierten bzw. vom Objekt auf den Spiegel 4 zurückgesandten Strahlen 10 verlaufen in der Z-Richtung. Die Drehachse bzw. der Drehpunkt des zweiten Spiegels 4 bzw. des zweiten Scanners 2 liegt in der Mitte zwischen der Drehachse 5 und dem Drehpunkt des Spiegels 3. Es sind hier auch die Strahlen 10 angedeutet, welche vom Spiegel 4 durch eine Abbildungsoptik oder ein Fokuminierelement 16 entlang einer optischen Achse 12 zum Objekt 20 ausgesandt bzw. wieder reflektiert werden. Die von der Auskopplungseinrichtung zum Spiegel 3 des ersten Scanners gelangenden bzw. von jenem reflektierten Strahlen 8 verlaufen orthogonal nach vorn vor die Zeichenebene.

Die vom Spiegel 4 reflektierten Strahlen gelangen durch das Fokussierelement 16 entlang der optischen Achse 12 auf das Objekt 20, wobei der beleuchtende Laserstrahl aufgrund der periodischen und synchronen Bewegung der beiden Spiegel 3, 4 in den beiden Dimensionen senkrecht zur optischen Achse 12 abgelenkt wird. Obgleich das beschriebene Gerät in besonders zweckmäßiger Weise zur Durchführung des erfindungsgemäßen Verfahrens zum Einsatz gelangt, zumal es bei kompaktem Aufbau eine fehlerfreie Abbildung des Objektes gewährleistet, können im Rahmen der Erfindung auch andere Laser-Scanning-Systeme zum Einsatz gelangen.

Fig. 3 zeigt den grundsätzlichen Aufbau des erfindungsgemäßen Gerätes mit dem Laser-Scanning-System 30, welches mittels eines Computers 32 und einer Kontroll-und Steuerelektronik 34 betrieben wird. Nachfolgend wird zur Verdeutlichung der Zusammenhänge auf die Komponenten des Gerätes der Fig. 1 und 2 Bezug genommen. Der zweite Spiegel 4 oszilliert mit hoher Frequenz f und bewegt den Laserstrahl entlang einer Linie des untersuchten Objekts. Die Intensität des während der Abtastung dieser Linie reflektierten Lichts wird mit dem Detektor 26 in festen Zeitabständen gemessen, so daß sich entlang der abgetasteten Linie eine Reihe von M-Meßwerten ergibt, welche die reflektierten Lichtintensitäten an M-individuellen Punkten entlang dieser abgetasteten Linie wiedergibt. Die genannten M-Meßwerte werden digitalisiert und in dem Computer 32 gespeichert. Die Abtastung entlang dieser ersten Linie des Objektes wird Nmal nacheinander wiederholt, so daß jeder der M-Punkte entlang der Linie in der Anzahl N in gleichen zeitlichen Abständen von 1/f gemessen wird. Somit wird eine Matrix von M x N Meßwerten erfaßt. Diese Orts-Zeit-Matrix enthält in jeder ihrer N-Zeilen örtlich aufgelöst und in jeder ihrer M-Spalten zeitlich aufgelöst die reflektierte Lichtintensität an den einzelnen Punkten entlang der abgetasteten ersten Linie des Objektes.

Nach Aufnahme der Orts-Zeit-Matrix für die eine erste Linie des Objekts wird der abtastende Laserstrahl mittels des ersten Spiegels 3 der Strahlablenkungseinheit senkrecht zur Richtung der abgetasteten Linie auf eine benachbarte zweite Linie des Objektes verschoben. Der oben beschriebene Meßvorgang wird für diese zweite Linie wiederholt. Nachfolgend werden in entsprechender Weise weitere parallele Linien des Objektes abgetastet und es ergeben sich somit für L Linien entsprechend L Matrizen mit jeweils M x N Meßwerten. Folglich wird erfindungsgemäß ein zweidimensionales Feld des Objektes in M x L Punkten abgetastet und für jeden dieser Punkte liegt eine Folge von N Meßwerten in gleichen zeitlichen Abständen vor. Durch diese erfindungswesentliche Abfolge einerseits der räumlichen und andererseits der zeitlichen Abtastvorgänge wird es möglich, den erforderlichen Bereich der Fließgeschwindigkeit abzudecken und effiziente numerische Verfahren zur Spektralanalyse heranzuziehen. Die hohe Abtastgeschwindigkeit bedingt eine sehr hohe Effizienz bei der Detektion des reflektierten Lichtes, insbesondere mittels eines hochempfindlichen Detektors. Der anhand der Fig. 1 und 2 erläuterte optische Aufbau des Abtastsystems gewährleistet vor allem aufgrund der geringen Anzahl optischer Komponenten eine hohe Lichtausbeute und ferner die hochempfindliche Detektion, welche insbesondere mittels einer Avalanche-Fotodiode realisiert wird.

In einer typischen Ausgestaltung zur Messung des retinalen Blutflusses werden beispielsweise folgende Werte vorgegeben:

Der zweite Spiegel 4 zur Abtastung einer Linie entlang der Retina oszilliert mit einer Frequenz f = 8000 Hz. Für die Faktoren M, N, L wird jeweils der Wert 256 vorgegeben. Wie oben erläutert, wird somit ein Feld von L = 256 Zeilen zu jeweils M = 256 Punkten des Objektes abgetastet. Wird mit Hilfe der Abbildungsoptik des Fokussierelements die Länge der abgetasteten Linien zu im wesentlichen 3 mm gewählt, so wird ein örtlicher Abstand zwischen zwei Meßpunkten entlang einer Linie sowie zwischen zwei benachbarten Linien auf der Retina zu im wesentlichen 0,01 mm. Durch diesen Wert ist die räumliche Auflösung der Messung vorgegeben. Die Intensität des reflektierten Lichtes wird an jedem Punkt in der Anzahl L = 256 in festen Abständen von 1/f = 0,000125 Sekunden gemessen Die Datenaufnahmezeit für die Orts-Zeit-Matrix einer Zeile beträgt hierbei 1/f x L = 0,032 Sekunden und die gesamte Aufnahmezeit ist für die eingangs genannten Werte folglich 8 Sekunden. Insgesamt werden für die erläuterte typische Ausgestaltung der Messung 256 x 256 x 256 Meßwerte aufgenommen und digitalisiert.

Für die Auswertung der derart aufgenommenen und digitalisierten Daten wird davon ausgegangen, daß an jedem Punkt des untersuchten Objektes ein Teil des gemessenen Lichtes von beweglichen Komponenten des Objektes, insbesondere den Bestandteilen des fließenden Blutes, und ein anderer Teil von ortsfesten Objektteilen reflektiert wird. Aufgrund des Dopplereffekts verschiebt sich die Frequenz des an bewegten Teilen reflektierten Lichts relativ zur Frequenz des an unbewegten Teilen reflektierten Lichts. Infolge der Kohärenz des Laserlichts kommt es durch Überlagerung und Interferenz dieser beiden Komponenten am Detektor zu zeitlichen Schwankungen der an einem Punkt des Objektes gemessenen Intensität des reflektierten Lichts.

Der zeitliche Verlauf der jeweils an einem Punkt reflektierten Intensität ist gemäß obigen Darlegungen in einer diesem Punkt entsprechenden Spalte der Orts-Zeit-Matrix enthalten. Die Auswertung der zeitlichen Schwankungen führt zu der Berechnung der sie hervorrufenden Fließgeschwindigkeit an diesem Punkt. Hierzu wird erfindungsgemäß jede Spalte der Meßwert-Matrizen einer Frequenzanalyse unterworfen, wie z.B. der bekannten diskreten Fourier-Transformation, woraus die Frequenzverteilung der zeitlichen Schwankung der reflektierten Lichtintensität gebildet wird. Da eine bestimmte Fließgeschwindigkeit zu einer Intensitätsschwankung des gemessenen Lichtes mit einer bestimmten Frequenz führt, läßt sich aus der Frequenzverteilung die Geschwindigkeit bewegter Teile an dem jeweiligen Punkt des Objekts bstimmen. Die diskrete Fourier-Transformation der Meßwerte kann im Rahmen dieser Erfindung sowohl durch Software als auch durch spezielle Hardware des Computers realisiert sein.

Nach der erläuterten Berechnung der typischen Fließgeschwindigkeit an jedem Punkt des abgetasteten zweidimensionalen Felds des Objekts ergibt sich eine Matrix von M x L Geschwindigkeiten. Diese Matrix läßt sich insbesondere als Bild sichtbar machen, welches die Fließgeschwindigkeit örtlich aufgelöst wiedergibt. Für die oben erläuterte typische Ausgestaltung und die dort angegebenen Werte ergeben sich folgende Begrenzungen für die Messung der Fließgeschwindigkeit:

Die Grenzfrequenz bei der diskreten Fourier-Transformation beträgt f/2, also 4000 Hz im genannten Beispiel. Ein sich mit der Geschwindigkeit v entlang der Ausbreitung des Lichts, also parallel zur optischen Achse, bewegendes Objekt, ruft eine Frequenzverschiebung des reflektierten Lichts und damit eine Modulation der gemessenen Lichtintensität mit einer Frequenz $F = 2v/\lambda$ hervor, wobei $\lambda$ der Lichtwellenlänge entspricht. Für die genannten Werte ist somit eine maximale Fließgeschwindigkeit von 2 mm/s meßbar.

Besondere Ausgestaltungen des erfindungsgemäßen Verfahrens und/oder Gerätes werden nachfolgend erläutert. So kann das Laser-Scanning-System insbesondere als konfokales optisches System ausgebildet sein, wobei in bekannter Weise die Detektionseinrichtung für das reflektierte Licht im wesentlichen punktförmig ausgebildet ist. Hierfür wird beispielsweise vor dem Detektor eine kleine Blende angeordnet, welche sich in einer optisch konjugiert zur Fokalebene des Abtastsystems gelegenen Position befindet. Hierdurch wird sichergestellt, daß im wesentlichen nur dasjenige Licht detektiert wird, welches aus einer schmalen Umgebung der jeweils vorgegebenen Fokalebene reflektiert wird. Hingegen wird an anderen Stellen reflektiertes oder gestreutes Licht aufgrund der konfokalen Ausbildung wirkungsvoll unterdrückt. Es wird eine hohe optische Auflösung des Systems nicht nur senkrecht zur optischen Achse, sondern auch parallel zu dieser erreicht. Somit ist es möglich, die Fließgeschwindigkeit selektiv in einzelnen Schichten des Objekts, beispielsweise am Gefäßsystem der Netzhaut, zu messen und es lassen sich ferner die Fließgeschwindigkeiten im retinalen Gefäßbett sowie im Gefäßsystem der Aderhaut voneinander getrennt und dreidimensional darstellen.

In einer weiteren besonderen Ausgestaltung ist das optische System polarisationsempfindlich ausgelegt. Es wird hierzu eine linear polarisierte Laserquelle, insbesondere eine Laserdiode, verwendet oder unpolarisiertes Laserlicht wird mittels eines Polarisators linear polarisiert. Die gemäß Fig. 1 zur Auskopplung vorgesehene Einrichtung 22 wird ferner polarisations-empfindlich derart ausgebildet, daß nur solches reflektiertes Licht, welches in einer zum beleuchtenden Strahl um 90° gedrehten Richtung linear polarisiert ist, zum Detektor 26 gelangt. In einer solchen Anordnung wird die Detektion des vom Objekt unter Erhaltung der Polarisationsrichtung direkt reflektierten Lichts wirkungsvoll unterdrückt und es wird im wesentlichen nur gestreutes Licht detektiert. Alternativ wird mittels eines Viertel-Wellenlängen-Plättchens, welches an einer Stelle des Strahlenganges zwischen dem untersuchten Objekt 20 und der Auskoppeleinrichtung 22 angeordnet ist, die Polarisationsrichtung des reflektierten Lichtes im Vergleich zur Polarisationsrichtung der Laserquelle um 90° gedreht. Folglich wird im Zusammenwirken mit der polarisations-empfindlichen Auskoppeleinrichtung 22 im wesentlichen das Licht detektiert, welches unter Erhaltung der Polarisationsrichtung vom Objekt 20 reflektiert wurde. Durch Selektion einerseits des gestreuten oder andererseits des direkt reflektierten Lichtes werden weitere Aufschlüsse über die Verteilung der Fließgeschwindigkeit erhalten.

In einer anderen Ausführungsform der Erfindung wird die Variation der Wellenlänge des Laserlichts genutzt, und zwar insbesondere durch Einbau zweier unterschiedlicher Laser in das Gerät, wodurch die Erfassung unterschiedlicher Bereiche des Gefäßsystems ermöglicht wird. Es wird hierbei davon ausgegangen, daß Licht unterschiedlicher Wellenlänge eine unterschiedliche Eindringtiefe in das retinale Gewebe aufweist. Das retinale Pigment epithel, welches das retinale Gefäßsystem vom Aderhautgefäßbett trennt, ist undurchlässig für sichtbares Licht, jedoch transparent für infrarotes Licht. Bei Verwendung von sichtbarem Laserlicht wird im Rahmen dieser Erfindung nur Licht vom Gewebe oberhalb des retinalen Pigmentepithels reflektiert und der Blutfluß wird selektiv im retinalen Gefäßsystem gemessen. Durch Verwendung von Licht im nahen Infrarotbereich wird hingegen auch das Gefäßbett mit der Aderhaut erreicht und die gemessene Fließgeschwindigkeit ist eine Überlagerung aus dem Blutfluß in der Retina und dem Blutfluß in der Aderhaut. Durch erfindungsgemäße Subtraktion des bei sichtbarem Licht gewonnenen Blutflußbildes von dem bei infrarotem Licht gewonnen ergibt sich der Blutfluß der Aderhaut allein. Ferner wird die Tiefenlokalisation mit dem oben beschriebenen konfokalen Aufbau erreicht.

Schließlich sei festgehalten, daß die erfindungsgemäße Messung von Fließgeschwindigkeiten im zeitlichen Verlauf auch die Erfassung von zeitlichen Änderungen der Fließgeschwindigkeiten im Gefäßsystem ermöglicht.

Durch Sychronisation der Datenerfassung auf den Herzschlag und zeitlich aufgelöste Darstellung der retinalen Durchblutung über mehrere Herzschläge wird in besonderes zweckmäßiger Weise die Pulswellenlaufgeschwindigkeit innerhalb des retinalen Gefäßsystems gemessen. So können zuverlässig Heterogenitäten der Pulswellenausbreitung und/oder des Gefäßwiderstands dargestellt werden.

Für den zweiten schnell bewegten Spiegel (gemäß Fig. 1, Spiegel 4) der Strahlablenkungseinheit, wobei dieser Spiegel den Laserstrahl entlang einer Linie über das Objekt bewegt, kann grundsätzlich ein Polygonspiegel oder ein oszillierender Drehspiegel verwendet werden. Im letzteren Fall ergibt sich nach jeder hinlaufenden Bewegung des Spiegels zur Abtastung einer Objektlinie eine Totzeit zur Rückbewegung des Spiegels in seine Ausgangsposition. Erfindungsgemäß wird die Rücklaufzeit des Spiegels ebenfalls zur Gewinnung von Daten genutzt. Hierdurch entsteht für jede abgetastete Linie des Objekts eine zweite Orts-Zeit-Matrix von M x N Meßwerten, welche zur ersten Orts-Zeit-Matrix zeitlich verschoben ist. Diese beiden Orts-Zeit-Matrizen werden getrennt voneinander der Fourier-Transformation unterzogen und anschließend unter Berücksichtigung des Verschiebungssatzes der Fourier-Transformation zu einem Gesamtspektrum kombiniert. Durch diese besondere Ausgestaltung wird eine Verbesserung des Signal- und Rausch-Verhältnisses erreicht.

Für die Auswertung der Frequenzspektren, welche sich durch Fourier-Transformation aus den Orts-Zeit-Matrizen ergeben, bestehen im Rahmen dieser Erfindung verschiedene alternative Ausgestaltungen. Die Erfassung der bei einer starken Strömung vorhandenen charakteristischen Grenzfrequenz ermöglicht die Messung der höchsten vorkommenden Fließgeschwindigkeit. Die Bestimmung des Schwerpunkts des Frequenzspektrums oder die Bestimmung der Frequenz, deren Wert der spektralen Leistungsdichte dem arithmetischen Mittel des Gesamtspektrums entspricht, ermöglicht die Bestimmung mittlerer Fließgeschwindigkeiten. Ferner erlaubt der Vergleich der spektralen Leistungsdichten einzelner Frequenzen, die Bestimmung der relativen Häufigkeit unterschiedlicher Fließgeschwindigkeiten.

Eine besondere Art der Auswertung der Frequenzspektren ist die Bandpaßmethode. Bei dieser Methode wird der Mittelwert der spektralen Leistungsdichte in einem bestimmten Frequenzintervall, welches einer bestimmten mittleren Fließgeschwindigkeit entspricht, berechnet und hieraus wird ein Blutflußbild dieses speziellen Geschwindigkeitsbereiches erzeugt. Durch unterschiedliche Mittenfrequenzen solcher Bandpässe werden gezielt langsame, beispielsweise kapiläre, und schnelle, beispielsweise arterioläre, Strömungen untersucht. Die spektrale Breite des Durchlaßbereiches der Bandpässe ist dabei ein Maß für den jeweils erfaßten Frequenz- und damit Geschwindigkeitsbereich.

Nach einer weiteren Ausgestaltung der Erfindung kann das beschriebene Gerät und/oder Verfahren zur Laser-Doppler-Flowmetrie eingesetzt werden. Bei dieser Ausgestaltung wird an jedem Punkt des untersuchten Gebietes die gemessene Fließgeschwindigkeit mit dem Verhältnis der von unbewegten und bewegten Komponenten reflektierten Lichtintensität multipliziert, welches sich aus der Anlyase der Frequenzspektren ergibt. Hierdurch wird in besonders zweckmäßiger Weise ein örtlich aufgelöstes Bild des Gesamtflusses erhalten.

**Bezugszeichen**

| 1 | erster Scanner |
|---|---|
| 2 | zweiter Scanner |
| 3 | erster Spiegel |
| 4 | zweiter Spiegel |
| 5, 6 | Achse |
| 7 | Abstand |
| 8, 9, 10 | Strahlen |
| 11 | Arm |
| 12 | optische Achse |
| 13, 14 | Linse |
| 16 | Fokussierelement |
| 20 | Objekt |
| 22 | Einrichtung |
| 24 | Strahlenquelle |
| 26 | Detektor |
| 30 | Laser-Scanning-System |
| 32 | Computer |
| 34 | Kontroll- und Steuerelektronik |
| M | Meßwerte entlang einer Linie |
| N | Anzahl der Messungen pro Linie |
| L | Anzahl der abgetasteten Linien |

**Patentansprüche**

1. Verfahren zur Messung der Fließgeschwindigkeit einer Flüssigkeit, insbesondere des Blutes, durch Bestimmung einer Frequenzverschiebung eines in der Flüssigkeit reflektierten Laserstrahls gemäß dem optischen Doppler-Effekt, wobei mittels des Laserstrahls eine zweidimensionale rasterförmige Abtastung eines Objekts in zueinander insbesondere parallelen Linien durchgeführt wird und aus der zeitlichen Variation der gemessenen Intensität des reflektierten Lichts die Doppler-Verschiebung berechnet und hieraus die Fließgeschwindigkeit bestimmt wird, wobei entlang einer abgetasteten Linie die reflektierten Lichtintensitäten einer Anzahl von M-Meßwerten erfaßt und gespeichert werden, dadurch gekennzeichnet, daß die Abtastung entlang der ersten Linie N mal nacheinander durchgeführt wird, wobei N eine ganze Zahl gleich oder größer 2 ist und an jedem Abtastpunkt entsprechend dem reflektierten Licht eine Anzahl N Meßwerte gebildet werden und wobei für diese Linie eine Matrix von M x N Meßwerten erfaßt wird, und daß nachfolgend für die zweite und weiteren Linien jeweils nacheinander diese pro Punkt N-fache Abtastung wiederholt wird.

2. Verfahren nach Anspruch 1, dadurch gekennzeichnet, daß die Abtastung in wenigstens zwei Ebenen in unterschiedlicher Tiefe eines Objektes durchgeführt wird, wobei ein Laser-Scanning-System in konfokaler Anordnung zum Einsatz gelangt und wobei die Abtastung und Messung in wenigstens zwei unterschiedlichen Fokalebenen durchgeführt wird.

3. Verfahren nach Anspruch 1 oder 2, dadurch gekennzeichnet, daß durch die Wahl der Wellenlänge des Laserlichtes die Messung in unterschiedlichen Bereichen des Objektes durchgeführt wird, wobei bevorzugt zwei unterschiedliche Laserquellen zum Einsatz gelangen.

4. Verfahren nach einem der Ansprüche 1 bis 3, dadurch gekennzeichnet, daß während der Abtastung einer Linie die Intensität des reflektierten Lichtes in festen Zeitabschnitten gemessen wird und daß die Abtastung der jeweiligen Linie in gleichen zeitlichen Abständen N mal nacheinander durchgeführt wird.

5. Verfahren nach einem der Ansprüche 1 bis 4, dadurch gekennzeichnet, daß die pro abgetastete Linie erfaßte Matrix von M x N Meßwerten, enthaltend N Zeilen der örtlich aufgelösten sowie M Spalten der zeitlich aufgelösten reflektierten Lichtintensität einer Spektralanalyse, insbesondere einer diskreten Fourier-Transformation unterworfen wird und hieraus die Frequenzverteilung der zeitlichen Schwankung der reflektierten Lichtintensität bestimmt wird, und daß aus der derart bestimmten Frequenzverteilung die Geschwindigkeitsverteilung der bewegten Teile der Flüssigkeit an dem jeweiligen Punkt des Objektes bestimmt wird.

6. Verfahren nach einem der Ansprüche 1 bis 5, dadurch gekennzeichnet, daß nach der Berechnung der typischen Fließgeschwindigkeit an jedem Punkt des abgetasteten zweidimensionalen Feldes des Objektes eine Matrix von M x L Geschwindigkeiten bestimmt wird, welche insbesondere nach Sichtbarmachung in einem Bild, örtlich aufgelöst die Fließgeschwindigkeit wiedergibt, wobei L die Anzahl der abgetasteten Linien bedeutet und eine ganze Zahl gleich oder größer 2 ist.

7. Verfahren nach einem der Ansprüche 1 bis 6, dadurch gekennzeichnet, daß bei der Messung der Fließgeschwindigkeit des Blutes die Erfassung der Meßwerte auf den Herzschlag synchronisiert wird.

8. Gerät zur Messung der Fließgeschwindigkeit einer Flüssigkeit mit einem Laser-Abtastsystem mit einer Strahlablenkungseinheit (1, 2) zur periodischen Ablenkung des Laserstrahls in zwei zueinander senkrechten Richtungen, einem Computer (32) zur Analyse der gewonnenen Meßdaten und einer Kontroll- und Steuereinrichtung, dadurch gekennzeichnet, daß die Kontroll- und Steuereinrichtung geeignet ist, die Abtastung entlang einer ersten Linie N mal nacheinander durchzuführen, wobei N eine ganze Zahl gleich oder größer 2 ist, so daß an jedem Abtastpunkt entsprechend dem reflektierten Licht eine Anzahl N Meßwerte gebildet werden, und nachfolgend für die zweite und weiteren Linien jeweils nacheinander diese pro Punkt N-fache Abtastung zu wiederholen.

9. Gerät nach Anspruch 8, dadurch gekennzeichnet, daß eine Abbildungsoptik zur Abbildung des abtastenden Laserstrahls auf das zu untersuchende Objekt (20) und/oder ein Fokussierelement zur Einstellung der Fokalebene vorgesehen ist, wobei zur Ablenkung der Strahlen zwei periodisch und synchron bewegte Spiegel (3, 4) zur Ablenkung des beleuchtenden Laserstrahls in zwei Dimensionen orthogonal zur optischen Achse (12) vorgesehen sind.

10. Gerät nach Anspruch 8 oder 9, dadurch gekennzeichnet, daß der zweite Spiegel (4) mit hoher Frequenz (f) oszilliert und den Laserstrahl entlang einer Linie des untersuchten Objektes (20) bewegt, wobei die Abtastung entlang der jeweiligen Linie des Objektes N mal erfolgt und daß ein Detektor (26) vorgesehen ist, mittels welchem entlang der jeweiligen Linie für jeden der M Punkte die N-fache Abtastung in gleichen zeitlichen Abständen von 1/f erfolgt.

11. Gerät nach einem der Ansprüche 8 bis 10, dadurch gekennzeichnet, daß der Mittelpunkt des zweiten Spiegels (4) in der Mitte des Abstandes (7) zwischen dem ersten Spiegel (3) und dessen Drehachse (5) angeordnet ist und daß die Strahlen vom ersten Spiegel (3) direkt zum zweiten Spiegel (4) und umgekehrt verlaufen und/oder daß der erste Spiegel (3) über einen Arm (11) mit seiner zugehörenden Drehachse gekoppelt ist, wobei die Länge des Armes (11) im wesentlichen gleich groß wie der genannte Abstand (7) ist.

12. Gerät nach einem der Ansprüche 8 bis 11, dadurch gekennzeichnet, daß der Computer (32) zur Speicherung einer Matrix von M x N digitalisierten Meßwerten ausgebildet ist, deren N-Zeilen örtlich aufgelöst und deren M-Spalten zeitlich aufgelöst der reflektierten Lichtintensität an den einzelnen Punkten entsprechen.

13. Gerät nach Anspruch 12, dadurch gekennzeichnet, daß der Computer (32) entsprechend der Anzahl L der abgetasteten Linien zur Speicherung und / oder Auswertung von L-Matrizen mit jeweils M x N Meßwerten ausgebildet ist.

14. Gerät nach Anspruch 12 oder 13, dadurch gekennzeichnet, daß ein Signalprozessor oder ein Hardware-mäßiger Fouriertransformator zur Durchführung einer Spektralanalyse vorgesehen ist.

15. Gerät nach einem der Ansprüche 8 bis 14, dadurch gekennzeichnet, daß ein Detektor (26) mit hoher Empfindlichkeit, insbesondere eine Avalanche-Fotodiode oder ein vergleichbarer hochempfindlicher Detektor vorgesehen ist.

16. Gerät nach einem der Ansprüche 8 bis 15, dadurch gekennzeichnet, daß das optische System polarisations-empfindlich ausgelegt ist, wobei insbesondere eine linear polarisierte Laserquelle zum Einsatz gelangt oder unpolarisiertes Laserlicht mittels eines Polarisators linear polarisiert wird, und daß die Auskoppeleinrichtung (22) gleichfalls polarisations-empfindlich derart ausgebildet ist, daß nur solches reflektiertes Licht, welches in einer zum beleuchtenden Strahl um im wesentlichen 90° gedrehten Richtung linear polarisiert ist, zum Detektor (26) reflektiert wird, und/oder bevorzugt durch ein zusätzlich zwischen dem untersuchten Objekt (20) und der Auskoppeleinrichtung (22) angeordneten 1/4-Wellenlängen-Plättchen die Polarisationsrichtung des reflektierten Lichtes im Vergleich zur Polarisationsrichtung der Laserquelle um 90° gedreht wird.

17. Gerät nach einem der Ansprüche 8 bis 16, dadurch gekennzeichnet, daß die Kontroll- und Steuereinrichtung derart ausgebildet ist, daß nach der Abtastung entlang einer Linie des Objektes während der Rücklaufzeit des zweiten Spiegels (4) für die jeweils abgetastete Linie eine zur ersten Orts-Zeit-Matrix zeitlich verschobene zweite Orts-Zeit-Matrix erfaßt wird, wobei die genannten beiden Orts-Zeit-Matrizen getrennt voneinander Fourier-transformiert und anschließend unter Berücksichtigung des Verschiebungssatzes der Fourier-Transformation zu einem Gesamtspektrum kombiniert werden, wodurch eine Verbesserung des Signal-/Rausch-Verhältnisses erzielt wird.

18. Gerät nach einem der Ansprüche 8 bis 17, dadurch gekennzeichnet, daß der Computer (32) zur Laser-Doppler-Flowmetrie derart ausgebildet ist, daß an jedem Punkt eines untersuchten Gebietes die gemessene Fließgeschwindigkeit mit dem Verhältnis der von unbewegten Komponenten und der von bewegten Komponenten reflektierten Lichtintensität multipliziert wird, wobei das Verhältnis aus der Analyse der Frequenzspektren gebildet wird.

## Claims

1. Method for measuring the flow rate of a fluid, in particular of the blood, by determination of a frequency shift of a laser beam reflected in the fluid in accordance with the optical Doppler effect, in which two-dimensional raster scanning of an object in lines which are in particular parallel to one another is carried out by means of the laser beam and the Doppler shift is calculated from the variation in time of the measured intensity of the reflected light and is used to determine the flow rate, the reflected light intensities of a number of M measured values along a scanned line being acquired and stored, characterized in that the scanning along the first line is carried out N times in succession, where N is an integer equal to or greater than 2 and a number of N measured values are formed at each scanning point corresponding to the reflected light and where a matrix of M x N measured values is acquired for this line, and in that, subsequently, this N-times scanning per point is repeated for the second and further lines in each case in succession.

2. Method according to Claim 1, characterized in that the scanning is carried out in at least two planes at different depths of an object, a laser scanning system in confocal arrangement being employed and the scanning and measurement being carried out in at least two different focal planes.

3. Method according to Claim 1 or 2, characterized in that, by choosing the wavelength of the laser light, the measurement is carried out in different regions of the object, preferably two different laser sources being employed.

4. Method according to one of Claims 1 to 3, characterized in that, during the scanning of a line, the intensity of the reflected light is measured at fixed time periods and in that the scanning of the respective line is carried out at equal time intervals N-times in succession.

5. Method according to one of Claims 1 to 4, characterized in that the matrix of M x N measured values acquired per scanned line, containing N rows of the locally resolved reflected light intensity and M columns of the temporally resolved reflected light intensity, is subjected to a spectral analysis, in particular a discrete Fourier transform, and this is used to determine the frequency distribution of the fluctuation in time of the reflected light intensity, and in that the frequency distribution determined in this way is used to determine the velocity distribution of the moving parts of the fluid at the respective point of the object.

6. Method according to one of Claims 1 to 5, characterized in that, after the calculation of the typical flow rate at each point of the scanned two-dimensional field of the object, a matrix of M x L velocities is determined, which, in particular after visualization in an image, represents the flow rate locally resolved, L signifying the number of scanned lines and being an integer equal to or greater than 2.

7. Method according to one of Claims 1 to 6, characterized in that, during the measurement of the flow rate of the blood, the acquisition of the measured values is synchronized with the heartbeat.

8. Apparatus for measuring the flow rate of a fluid using a laser scanning system having a beam deflection unit (1, 2) for the periodic deflection of the laser beam in two mutually perpendicular directions, a computer (32) for the analysis of the measured data obtained and a control device, characterized in that the control device is suitable for carrying out the scanning along a first line N times in succession, where N is an integer equal to or greater than 2, so that a number of N measured values are formed at each scanning point corresponding to the reflected light, and, subsequently, for repeating this N-times scanning per point for the second and further lines in each case in succession.

9. Apparatus according to Claim 8, characterized in that provision is made for imaging optics for projecting the scanning laser beam onto the object (20) to be investigated and/or a focusing element for adjusting the focal plane, there being provided, for the deflection of the beams, two periodically and synchronously moving mirrors (3, 4) for the deflection of the illuminating laser beam in two dimensions orthogonal to the optical axis (12).

10. Apparatus according to Claim 8 or 9, characterized in that the second mirror (4) oscillates at a high frequency (f) and moves the laser beam along a line of the object (20) being investigated, the scanning along the respective line of the object being performed N times, and in that provision is made for a detector (26) by means of which the N-times scanning is performed at equal time intervals of 1/f for each of the M points along the respective line.

11. Apparatus according to one of Claims 8 to 10, characterized in that the centre point of the second mirror (4) is arranged at the middle of the distance (7) between the first mirror (3) and the axis of rotation (5) of the latter and in that the beams proceed from the first mirror (3) directly to the second mirror (4) and vice versa and/or in that the first mirror (3) is coupled via an arm (11) to its associated axis of rotation, the length of the arm (11) being substantially equal in size to the said distance (7).

12. Apparatus according to one of Claims 8 to 11, characterized in that the computer (32) is designed to store a matrix of M x N digitized measured values, the N rows of which correspond, locally resolved, to the reflected light intensity at the individual points and the M columns of which correspond, temporally resolved, to the reflected light intensity at the individual points.

13. Apparatus according to Claim 12, characterized in that the computer (32) is designed to store and/or evaluate L matrices of M x N measured values each, in accordance with the number L of scanned lines.

14. Apparatus according to Claim 12 or 13, characterized in that provision is made for a signal processor or a hardware Fourier transformer for carrying out a spectral analysis.

15. Apparatus according to one of Claims 8 to 14, characterized in that provision is made for a detector (26) of high sensitivity, in particular an avalanche photodiode or a comparable high-sensitivity detector.

16. Apparatus according to one of Claims 8 to 15, characterized in that the optical system is designed to be polarization-sensitive, a plane-polarized laser source being employed in particular or nonpolarized laser light being plane-polarized by means of a polarizer, and in that the decoupling device (22) is likewise designed to be polarization-sensitive such that only that reflected light which is plane-polarized in a direction turned through substantially 90° with respect to the illuminating beam is reflected to the detector (26), and/or preferably a 1/4-wavelength lamina additionally arranged between the object (20) being investigated and the decoupling device (22) is used to turn the polarizing direction of the reflected light through 90° in comparison with the polarizing device of the laser source.

17. Apparatus according to one of Claims 8 to 16, characterized in that the control device is designed such that, after the scanning along a line of the object, a second local/temporal matrix deferred with respect to the first local/temporal matrix is acquired for the respectively scanned line during the return time of the second mirror (4), the said two local/temporal matrices being Fourier transformed separately from one another and subsequently being combined to give an overall spectrum while taking account of the displacement law of the Fourier transform, thereby obtaining an improvement in the signal-to-noise ratio.

18. Apparatus according to one of Claims 8 to 17, characterized in that the computer (32) is designed for laser doppler flowmetry such that, at each point of a region being investigated, the measured flow rate is multiplied by the ratio of the light intensity reflected by nonmoving components to that reflected by moving components, the ratio being formed from the analysis of the frequency spectra.

**Revendications**

1. Procédé de mesure de la vitesse d'écoulement d'un fluide, en particulier du sang, par la détermination d'un décalage de fréquence d'un rayon laser réfléchi dans le liquide suivant l'effet Doppler optique, un balayage bidimensionnel en réseau d'un objet en lignes en particulier parallèles étant effectué au moyen du rayon laser et le décalage Doppler étant calculé à partir de la variation temporelle de l'intensité de lumière réfléchie mesurée et la vitesse d'écoulement en étant ensuite déduite, les intensités lumineuses réfléchies le long d'une ligne balayée étant relevées et enregistrées pour une pluralité de M valeurs de mesure, *caractérisé en ce que* le balayage le long de la première ligne est effectué N fois successivement, N étant un nombre entier supérieur ou égal à 2 et un nombre N de valeurs de mesure étant relevé en chaque point de balayage en fonction de la lumière réfléchie et, pour cette ligne, une matrice de M x N valeurs de mesure étant relevée, et *en ce que* ce balayage N fois par point est ensuite répété successivement pour la deuxième ligne et pour d'autres lignes les unes après les autres.

2. Procédé selon la Revendication 1, *caractérisé en ce que* le balayage est effectué dans au moins deux plans à des profondeurs différentes d'un objet, un système de balayage laser à agencement à foyer commun étant utilisé et le balayage et la mesure étant effectués dans au moins deux plans focaux différents.

3. Procédé selon la Revendication 1 ou 2, *caractérisé en ce que*, par le choix de la longueur d'onde de la lumière laser, la mesure est effectuée dans différentes zones de l'objet, deux sources laser différentes étant de préférence utilisées.

4. Procédé selon l'une quelconque des Revendications 1 à 3, *caractérisé en ce que*, pendant le balayage d'une ligne, l'intensité de la lumière réfléchie est mesurée à des intervalles de temps fixes et *en ce que* le balayage de la ligne respective est effectué N fois successivement à des intervalles de temps réguliers.

5. Procédé selon l'une quelconque des Revendications 1 à 4, *caractérisé en ce que* la matrice de M x N valeurs de mesures relevées pour chaque ligne balayée, qui comprend N lignes d'intensités lumineuses réfléchies réparties dans l'espace et M colonnes d'intensités lumineuses réfléchies réparties dans le temps, est soumis à une analyse spectrale, en particulier à une transformation discrète de Fourier, et la distribution de fréquences de la fluctuation temporelle de l'intensité lumineuse réfléchie en est déterminée, et *en ce qu'*à partir de la distribution de fréquences ainsi déterminée, la distribution de vitesses des parties mobiles du liquide en chaque point de l'objet est déterminée.

6. Procédé selon l'une quelconque des Revendications 1 à 5, *caractérisé en ce qu'*après le calcul de la vitesse d'écoulement typique en chaque point de la zone bidimensionnelle balayée de l'objet, on détermine une matrice de M x L vitesses qui, en particulier après représentation sous forme d'image, reflète la distribution spatiale de la vitesse d'écoulement, L représentant le nombre des lignes balayées et étant un nombre entier supérieur ou égal à 2.

7. Procédé selon l'une quelconque des Revendications 1 à 6, *caractérisé en ce que*, lors de la mesure de la vitesse d'écoulement du sang, le relevé des valeurs de mesures est détecté avec le pouls cardiaque.

8. Appareil pour mesurer la vitesse d'écoulement d'un liquide avec un système de balayage laser avec une unité de déviation de rayon (1, 2) afin de dévier périodiquement le rayon laser dans deux directions mutuellement perpendiculaires, un ordinateur (32) pour l'analyse des valeurs de mesures collectées, et une unité de contrôle et de commande, *caractérisé en ce que* l'unité de contrôle et de commande convient pour effectuer le balayage le long d'une première ligne N fois à la suite, N étant un nombre entier supérieur ou égal à 2, de sorte qu'en chaque point de balayage, un nombre N de valeurs de mesure est relevé en fonction de la lumière réfléchie, et ce balayage N fois par point est ensuite répété successivement pour la deuxième ligne et pour d'autres lignes les unes après les autres.

9. Appareil selon la Revendication 8, *caractérisé en ce qu'*une optique de reproduction pour reproduire le rayon laser de balayage sur l'objet (20) à examiner et/ou un élément de focalisation pour le réglage du plan focal est prévu, deux miroirs (3, 4) mobiles périodiquement et de manière synchrone étant prévus pour la déviation des rayons, afin de dévier le rayon laser incident dans deux dimensions orthogonalement à l'axe optique (12).

10. Appareil selon la Revendication 8 ou 9, *caractérisé en ce que* le deuxième miroir (4) oscille à une fréquence élevée (f) et déplace le rayon laser le long d'une ligne de l'objet (20) à examiner, le balayage se faisant N fois le long de la ligne respective de l'objet, et *en ce qu'*il est prévu un détecteur (26) au moyen duquel, le long de la ligne respective, pour chacun des M points s'effectue le balayage N fois à des intervalles de temps réguliers de 1/f.

11. Appareil selon l'une quelconque des Revendications 8 à 10, *caractérisé en ce que* le centre du deuxième miroir (4) est placé au milieu de la distance (7) entre le premier miroir (3) et son axe de rotation (5) et *en ce que* les rayons vont directement du premier miroir (3) au deuxième miroir (4) et inversement et/ou *en ce que* le premier miroir (3) est couplé par un bras (11) à son axe de rotation associé, la longueur du bras (11) étant sensiblement égale à ladite distance (7).

12. Appareil selon l'une quelconque des Revendications 8 à 11, *caractérisé en ce que* l'ordinateur (32) est conformé pour enregistrer une matrice de M x N valeurs de mesure numérisées, dont les N lignes représentent les intensités lumineuses réfléchies réparties dans l'espace et les M colonnes représentent les intensités lumineuses réfléchies réparties dans le temps aux différents points.

13. Appareil selon la Revendication 12, *caractérisé en ce que* l'ordinateur (32) est conformé en fonction du nombre L de lignes balayées pour enregistrer et/ou analyser L matrices de chacune M x N valeurs de mesure.

14. Appareil selon la Revendication 12 ou 13, *caractérisé en ce qu'*un processeur de signaux ou un transformateur de Fourier de type matériel est prévu pour effectuer une analyse spectrale.

15. Appareil selon l'une quelconque des Revendications 8 à 14, *caractérisé en ce qu'*un détecteur (26) de grande sensibilité, en particulier une photodiode à avalanche ou un détecteur comparable de grande sensibilité, est prévu.

16. Appareil selon l'une quelconque des Revendications 8 à 15, *caractérisé en ce que* le système optique est conformé sensible à la polarisation, en particulier une source de laser polarisée linéairement étant utilisée ou une lumière laser non polarisée étant polarisée linéairement au moyen d'un polariseur, et *en ce que* le dispositif de découplage (22) est également conformé sensible à la polarisation, de manière que seule la lumière réfléchie qui est polarisée linéairement dans une direction tournée sensiblement de 90° par rapport au rayon incident soit réfléchie vers le détecteur (26), et/ou de préférence au moyen d'une lame quart d'onde placée en un point de la trajectoire des rayons entre l'objet examiné (20) et le dispositif de découplage (22), la direction de polarisation de la lumière réfléchie soit tournée de 90° par rapport à la direction de polarisation de la source laser.

17. Appareil selon l'une quelconque des Revendications 8 à 16, *caractérisé en ce que* le dispositif de contrôle et de commande est conformé de manière qu'après le balayage le long d'une ligne de l'objet, pendant le temps de retour du deuxième miroir (4), une deuxième matrice lieu-temps décalée dans le temps par rapport à la première matrice lieu-temps est relevée pour la ligne balayée, les deux matrices lieu-temps citées étant soumises séparément à une transformation de Fourier puis, en tenant compte du jeu de décalage de la transformation de Fourier, combinées en un spectre global, ce qui permet une amélioration du rapport signal/bruit.

18. Appareil selon l'une quelconque des Revendications 8 à 17, *caractérisé en ce que* l'ordinateur (32) est conformé pour une débitmétrie laser à effet Doppler, de sorte qu'en chaque point d'une zone examinée, la vitesse d'écoulement mesurée est multipliée par le rapport des intensités lumineuses réfléchies par les composantes immobiles et mobiles, le rapport résultant de l'analyse des spectres de fréquence.

FIG.1

FIG.2

FIG.3